(19) Europäisches Patentamt
European Patent Office
Office européen des brevets

(11) Veröffentlichungsnummer: **0 012 965**
**B1**

(12) # EUROPÄISCHE PATENTSCHRIFT

(45) Veröffentlichungstag der Patentschrift: **25.08.82**

(51) Int. Cl.³: **A 61 K 7/13**

(21) Anmeldenummer: **79105204.6**

(22) Anmeldetag: **17.12.79**

(54) **Mittel und Verfahren zur Färbung von Haaren.**

(30) Priorität: **23.12.78 DE 2855917**

(43) Veröffentlichungstag der Anmeldung: **09.07.80 Patentblatt 80/14**

(45) Bekanntmachung des Hinweises auf die Patenterteilung: **25.08.82 Patentblatt 82/34**

(84) Benannte Vertragsstaaten: **AT BE CH DE FR GB IT NL SE**

(56) Entgegenhaltungen:
**DE-A-2 628 999**
**DE-A-2 737 138**
**FR-A-2 112 550**

**Die Akte enthält technische Angaben, die nach dem Eingang der Anmeldung eingereicht wurden und die nicht in dieser Patentschrift enthalten sind.**

(73) Patentinhaber: **Wella Aktiengesellschaft, Berliner Allee 65, D-6100 Darmstadt (DE)**

(72) Erfinder: **Konrad, Eugen, Mecklenburger Strasse 101, D-6100 Darmstadt (DE)**
Erfinder: **Husemeyer, Hans, Dr., Thylmannweg 5, D-6100 Darmstadt (DE)**
Erfinder: **Mager, Herbert, Dr., Beaumont 5, CH-1700 Fribourg (CH)**

EP 0 012 965 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Mittel und Verfahren zur Färbung von Haaren

Gegenstand der Erfindung ist ein Mittel und ein Verfahren zur oxidativen Färbung von Haaren auf der Basis eines 1-(β-Hydroxyalkyl)-2,4-diaminobenzols als Kupplersubstanz.

Es sind bereits Mittel zum oxidativen Färben von Haaren beschrieben worden, die neben Oxydationsbasen wie p-Phenylendiaminen mindestens eine Kupplersubstanz der allgemeinen Formel

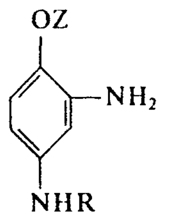

in der R ein Wasserstoffatom oder einen Alkyl- oder Hydroxyalkylrest mit 1 bis 3 Kohlenstoffatomen bedeutet und Z einen Hydroxyalkyl-, Alkoxyalkyl- (wobei der Alkoxyrest 1 oder 2 Kohlenstoffatome aufweist), Mesylaminoalkyl-, Acetylaminoalkyl-, Ureidoaminoalkyl- oder Carbähtoxyaminoalkylrest (wobei die Alkylreste in den Resten Z 2 oder 3 Kohlenstoffatome aufweisen) darstellt, sowie die Säuresalze dieser Verbindungen, enthalten. Als Kupplersubstanz kann insbesondere auch das 2,4-Diaminophenoxyäthanol in Betracht kommen. Derartige Färbemittel mit einem Gehalt an Kupplersubstanzen der obigen Formel führen bei Kombination mit einem Großteil der p-Phenylendiamine zu blauen Haarfärbungen, die mehr oder weniger reich an grünen oder purpurnen Tönen sind (vgl. DE-A-2 737 138).

Für das Färben von Haaren haben Färbemittel auf der Grundlage von Oxidationsfarbstoffen eine erhebliche Bedeutung erlangt. Die Färbung entsteht hierbei in der Weise, daß bestimmte Entwicklersubstanzen mit bestimmten Kupplersubstanzen in Gegenwart eines geeigneten Oxidationsmittels reagieren.

Als Entwicklersubstanzen werden vor allem 2,5-Diaminotoluol, 1,4-Diaminobenzol, p-Aminophenol und 3-Methyl-4-aminophenol eingesetzt. Vorzugsweise verwendete Kupplersubstanzen sind α-Naphthol, Resorcin, 4-Chlorresorcin, m-Aminophenol und Derivate des m-Phenylendiamins wie m-Toluylendiamin und 2,4-Diaminoanisol. Diese Derivate sowie das m-Phenylendiamin selbst haben hierbei wegen ihrer Fähigkeit, bei der oxidativen Kupplung mit 1,4-Diaminobenzol bzw. 1,4-Diaminobenzolderivaten Blautöne zu erzeugen, als sogenannte Blaukuppler Bedeutung erlangt.

An Oxidationsfarbstoffe, die zur Färbung menschlicher Haare Verwendung finden, sind zahlreiche besondere Anforderungen gestellt. So müssen sie in toxikologischer und dermatologischer Hinsicht unbedenklich sein und die Erzielung von Färbungen in der gewünschten Intensität ermöglichen. Weiterhin ist es erforderlich, daß durch Kombination geeigneter Entwickler- und Kupplerkomponenten eine breite Palette unterschiedlicher Farbnuancen erzeugt werden kann. Ferner wird für die erzielbaren Haarfärbungen eine gute Licht-, Dauerwell-, Säure- und Reibechtheit gefordert. Auf jeden Fall aber müssen solche Haarfärbungen ohne Einwirkung von Licht, chemischen Mitteln und Reibung über einen Zeitraum von mindestens 4 bis 6 Wochen stabil bleiben.

Das zur Zeit in Haarfärbemitteln als Blaukuppler verwendete m-Phenylendiamin, dessen Derivate m-Toluylendiamin und 2,4-Diaminoanisol sowie auch in neuerer Zeit empfohlene Blaukuppler, wie beispielsweise 1-Hydroxy-3-amino-6-chlorbenzol und 2,4-Diaminophenoxyäthanol, können die vorstehend genannten Anforderungen jedoch noch nicht zufriedenstellend erfüllen.

Hierzu wurde nun gefunden, daß Mittel zur oxidativen Färbung von Haaren mit einem Gehalt an mindestens einem 1-(β-Hydroxyalkyl)-2,4-diaminobenzol der allgemeinen Formel

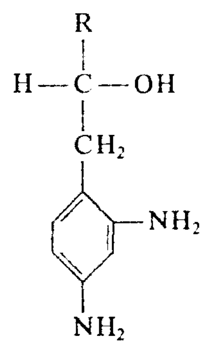

in der R ein Wasserstoffatom, eine $CH_3$- oder $C_2H_5$-Gruppe bedeutet, auch in Form seiner anorganischen oder organischen Salze, als Kupplersubstanz in besonders hohem Maße der gestellten Aufgabe gerecht werden.

Die als Kupplersubstanzen in den erfindungsgemäßen Haarfärbemitteln enthaltenen 1-(β-Hydroxyalkyl)-2,4-diaminobenzole der angegebenen Formel sind gut in Wasser löslich, wobei die Löslichkeit durch Zusatz von Alkalien wie beispielsweise Natriumhydroxid noch gesteigert werden kann, und weisen außerdem eine ausgezeichnete Lagerbeständigkeit, insbesondere als Bestandteil der erfindungsgemäßen Haarfärbemittel, auf.

In den Haarfärbemitteln sollen die genannten Kupplersubstanzen, von denen das 1-(b-Hydroxyäthyl)-2,4-diaminobenzol bevorzugt wird, in einer Konzentration von 0,01 bis 4,0 Gewichtsprozent, vorzugsweise 0,02 bis 2,0 Gewichtsprozent, enthalten sein.

Außerdem können die Haarfärbemittel zusätzlich bekannte Kupplersubstanzen, insbesondere α-Naphthol, 3,4-Diaminobenzoesäure, Resorcin, 4-Chlorresorcin, m-Aminophenol und 3-Amino-6-methylphenol, enthalten. Auch 4-Oxy-1,2-methy-

lendioxybenzol ist vorteilhaft als Kuppler verwendbar.

Von den bekannten Entwicklersubstanzen kommen als Bestandteil der erfindungsgemäßen Haarfärbemittel vor allem 1,4-Diaminobenzol, 2,5-Diaminotoluol, p-Aminophenol und 3-Methyl-4-aminophenol in Betracht. Ferner kann auch 2,5-Diaminobenzylalkohol als geeigneter Entwickler eingesetzt werden.

Sowohl die erfindungsgemäß verwendeten 1-(b-Hydroxyalkyl)-2,4-diaminobenzole als auch die bekannten Kuppler- und Entwicklersubstanzen können in den Haarfärbemitteln jeweils allein oder im Gemisch miteinander enthalten sein.

Die Gesamtmenge der in den hier beschriebenen Haarfärbemitteln enthaltenen Entwickler-Kupplersubstanz-Kombination soll 0,1 bis 5,0 Gewichtsprozent, vorzugsweise 0,5 bis 3,0 Gewichtsprozent, betragen.

Die Entwicklerkomponenten werden im allgemeinen in etwa äquimolaren Mengen, bezogen auf die Kupplerkomponenten, eingesetzt. Es ist jedoch nicht nachteilig, wenn die Entwicklerkomponente diesbezüglich in einem gewissen Überschuß oder Unterschuß vorhanden ist.

Weiterhin können die Haarfärbemittel der vorliegenden Anmeldung zusätzlich andere Farbkomponenten, zum Beispiel 6-Amino-3-methylphenol, sowie ferner übliche direktziehende Farbstoffe, beispielsweise Triphenylmethanfarbstoffe wie Diamond Fuchsin (C.I. 42 510) und Leather Ruby HF (C.I. 42 520), aromatische Nitrofarbstoffe wie 2-Nitro-1,4-diaminobenzol und 2-Amino-4-nitrophenol, Azofarbstoffe wie Acid Brown 4 (C.I. 14 805) und Acid Blue 135 (C.I. 13 385), Anthrachinonfarbstoffe wie Disperse Red 15 (C.I. 60 710) und Disperse Violet 1 (C.I. 61 100), außerdem 1,4,5,8-Tetraamino-anthrachinon und 1,4-Diamino-anthrachinon enthalten.

Außerdem können in den Haarfärbemitteln noch weitere übliche kosmetische Zusätze, beispielsweise Antioxidantien wie Ascorbinsäure oder Natriumsulfit, Parfümöle, Komplexbildner, Netzmittel, Emulgatoren, Verdicker, Pflegestoffe und andere vorhanden sein.

Die Zubereitungsform kann die einer Lösung, vorzugsweise einer Creme, eines Gels oder einer Emulsion sein. Ihre Zusammensetzung stellt eine Mischung der Farbstoffkomponenten mit den für solche Zubereitungen üblichen Bestandteilen dar. Als übliche Bestandteile von Cremes, Emulsionen oder Gelen kommen beispielsweise Netzmittel oder Emulgatoren aus den Klassen der anionischen, kationischen oder nichtionogenen oberflächenaktiven Substanzen wie Fettalkoholsulfate, Alkylsulfonate, Alkylbenzolsulfonate, Alkyltrimethylammoniumsalze, oxäthylierte Fettalkohole, oxäthylierte Nonylphenole, Fettsäurealkanolamide, ferner Verdicker wie höhere Fettalkohole, Stärke, Cellulosederivate, Paraffinöl und Fettsäuren sowie außerdem Pflegestoffe wie Lanolinderivate, Cholesterin und Pantothensäure in Betracht. Die erwähnten Bestandteile werden in den für solche Zwecke üblichen Mengen verwendet, z. B. die Netzmittel und Emulgatoren in Konzentrationen von 0,5 bis 30 Gewichtsprozent, während die Verdicker in einer Menge von 0,1 bis 25 Gewichtsprozent in den Zubereitungen enthalten sein können.

Je nach Zusammensetzung können die erfindungsgemäßen Färbemittel schwach sauer, neutral oder alkalisch reagieren. Insbesondere weisen sie einen pH-Wert im alkalischen Bereich zwischen 8,0 und 11,5 auf, wobei die Einstellung vorzugsweise mit Ammoniak erfolgt. Es können aber auch organische Amine, z. B. Monoäthanolamin oder Triäthanolamin, Verwendung finden.

Bei dem erfindungsgemäßen Verfahren zur oxidativen Färbung von Haaren vermischt man die Haarfärbemittel, welche eine Kombination von in der Haarfärbung bekannten Entwicklersubstanzen mit mindestens einem 1-($\beta$-Hydroxyalkyl)-2,4-diaminobenzol der angegebenen Formel als Kupplersubstanz sowie gegebenenfalls zusätzlich bekannte Kupplersubstanzen enthalten, kurz vor Gebrauch mit einem Oxidationsmittel und trägt dieses Gemisch auf das Haar auf. Als Oxidationsmittel zur Entwicklung der Haarfärbung kommt hauptsächlich Wasserstoffperoxid, beispielsweise als 6%ige wäßrige Lösung bzw. dessen Additionsverbindungen an Harnstoff, Melamin oder Natriumborat in Betracht. Man läßt das Gemisch bei 15 bis 50°C 10 bis 45 Minuten lang, vorzugsweise 30 Minuten lang, auf das Haar einwirken, spült sodann das Haar mit Wasser aus und trocknet. Gegebenenfalls wird im Anschluß an diese Spülung mit einem Shampoo gewaschen und eventuell mit einer schwachen organischen Säure, wie z. B. Zitronensäure oder Weinsäure, nachgespült.

Zur Herstellung der in den beschriebenen Haarfärbemitteln enthaltenen 1-($\beta$-Hydroxyalkyl)-2,4-diaminobenzole sind in der Literatur verschiedene Synthesewege bekannt. So kann man beispielsweise bei der Herstellung von 1-($\beta$-Hydroxyäthyl)-2,4-diaminobenzol von $\beta$-Phenyläthylalkohol ausgehen. Der $\beta$-Phenyläthylalkohol wird zum Schutz der OH-Gruppe zunächst acetyliert und anschließend nitriert. Durch katalytische Hydrierung der entstandenen 2,4-Dinitroverbindung und nachfolgende Entacetylierung ist das 1-($\beta$-Hydroxyäthyl)-2,4-diaminobenzol in hoher Ausbeute erhältlich.

Hinsichtlich der färberischen Möglichkeiten bieten die erfindungsgemäßen Haarfärbemittel je nach Art und Zusammensetzung der Farbkomponenten eine breite Palette verschiedener Farbnuancen, welche sich von blonden über braune, aschige, purpurne, violette bis hin zu blauen und schwarzen Farbtönen erstreckt. Hierbei zeichnen sich die erzielbaren Farbtöne durch ihre starke Farbintensität aus.

Von besonderer Bedeutung ist weiterhin der durch die Verwendung der 1-($\beta$-Hydroxyalkyl)-2,4-diaminobenzole der angegebenen Formel in den hier beschriebenen Haarfärbemitteln in toxikologischer und dermatologischer Hinsicht, z. B. gegenüber den bekannten Blaukupplern 2,4-Diaminotoluol, 2,4-Diaminoanisol und

1,3-Diaminobenzol, erzielte Fortschritt, welcher auf der am substituierten Benzolkern gebundenen Hydroxyalkylgruppe und der damit verbundenen Verminderung der Lipoidlöslichkeit beruht.

Die vorstehend angegebenen 1-(β-Hydroxyalkyl)-2,4-diaminobenzole lietern als Kupplersubstanzen in Kombination mit den Entwicklersubstanzen 1,4-Diaminobenzol und 1,4-Diaminobenzolderivaten relativ kalte, sehr intensive Blautöne ohne Rotanteil, wie sie mit den in Haarfärbemitteln üblicherweise eingesetzten Kupplersubstanzen, beispielsweise 2,4-Diaminotoluol, 2,4-Diaminoanisol und 2,4-Diaminophenoxyäthanol und 3-Amino-6-chlorphenol, nicht erzielt werden können.

Liefert nämlich der zur Erzeugung von Aschtönen unbedingt erforderliche Blaukuppler mit den vorstehend als Entwicklersubstanzen genannten 1,4-Diaminoverbindungen rot- oder violettstichige Blautöne, so wird die Erlangung von Aschtönen unmöglich oder sehr erschwert. Demgegenüber ist es infolge der vorteilhaften Eigenschaft der anmeldungsgemäßen 1-(β-Hydroxyalkyl)-2,4-diaminobenzole, Blautöne ohne Rotanteil zu bilden, nun problemlos möglich, die Haare in aschigen Naturtönen zu färben.

Die überlegenen färberischen Eigenschaften der Haarfärbemittel gemäß vorliegender Anmeldung zeigen sich weiterhin darin, daß mit den als Kupplersubstanzen enthaltenen 1-(β-Hydroxyalkyl)-2,4-diaminobenzolen auch dunkle Naturtöne, insbesondere ein Blauschwarz, erzeugt werden können.

Schließlich ist mit Hilfe dieser Haarfärbemittel auch eine Anfärbung von ergrautem, chemisch nicht vorgeschädigtem Haar problemlos und mit bisher nicht erreichter Deckkraft möglich.

In den nachstehenden Beispielen soll der Gegenstand der Erfindung näher erläutert werden.

## Beispiele

### Beispiel 1

Haarfärbemittel in Gelform

| | |
|---|---|
| 0,5 g | 1-(β-Hydroxyäthyl)-2,4-diaminobenzoldihydrochlorid |
| 0,5 g | 2,5-Diaminotoluolsulfat |
| 0,3 g | Ascorbinsäure |
| 1,0 g | Hydroxyäthylcellulose, hochviskos |
| 5,0 g | Laurylalkohol-diglykoläthersulfat, Natriumsalz (28%ige wäßrige Lösung) |
| 10,0 g | Ammoniak, 22%ig |
| 82,7 g | Wasser |
| 100,0 g | |

Man vermischt kurz vor Gebrauch 50 g dieses Haarfärbemittels mit 50 ml Wasserstoffperoxidlösung (6%ig) und läßt das Gemisch 30 Minuten lang bei 40°C auf blonde menschliche Haare einwirken. Danach wird mit Wasser gespült und getrocknet. Das Haar hat eine intensive Blaufärbung erhalten.

### Beispiel 2

Haarfärbemittel in Gelform

| | |
|---|---|
| 0,5 g | 1-(β-Hydroxyäthyl)-2,4-diaminobenzol-dihydrochlorid |
| 1,0 g | 1,4-Diaminobenzol |
| 0,5 g | Resorcin |
| 0,2 g | m-Aminophenol |
| 0,3 g | Ascorbinsäure |
| 15,0 g | Ölsäure |
| 7,0 g | Isopropanol |
| 10,0 g | Ammoniak, 22%ig |
| 65,5 g | Wasser |
| 100,0 g | |

50 g dieses Haarfärbemittels werden kurz vor dem Gebrauch mit 50 ml Wasserstoffperoxidlösung (6%ig) vermischt und das Gemisch anschließend auf blonde menschliche Haare aufgetragen. Nach einer Einwirkungszeit von 30 Minuten bei 40°C wird mit Wasser gespült und getrocknet. Das Haar ist in einem tief blauschwarzen Ton gefärbt.

### Beispiel 3

Haarfärbemittel in Cremeform

| | |
|---|---|
| 0,5 g | 1-(β-Hydroxyäthyl)-2,4-diaminobenzol-dihydrochlorid |
| 0,3 g | p-Aminophenol |
| 0,3 g | Natriumsulfit, wasserfrei |
| 15,0 g | Cetylalkohol |
| 3,5 g | Laurylalkohol-diglykoläthersulfat, Natriumsalz (28%ige wäßrige Lösung) |
| 3,0 g | Ammoniak, 22%ig |
| 77,4 g | Wasser |
| 100,0 g | |

50 g dieses Haarfärbemittels werden kurz vor dem Gebrauch mit 50 ml Wasserstoffperoxidlösung (6%ig) vermischt und das Gemisch anschließend auf blonde menschliche Haare aufgetragen. Nach einer Einwirkungszeit von 30 Minuten bei 40°C wird mit Wasser gespült und getrocknet. Das Haar hat eine Roséfärbung erhalten.

Alle in der vorliegenden Anmeldung angegebenen Prozentzahlen stellen Gewichtsprozente dar.

## Patentansprüche

1. Mittel zur oxidativen Färbung von Haaren mit einem Gehalt von 0,1 bis 5,0 Gewichtsprozent an einer Entwicklersubstanz-Kupplersubstanz-Kombination, dadurch gekennzeichnet, daß es als Kupplersubstanz 0,01 bis 4,0 Gewichtsprozent eines 1-(β-Hydroxyalkyl)-2,4-diaminobenzols der

allgemeinen Formel

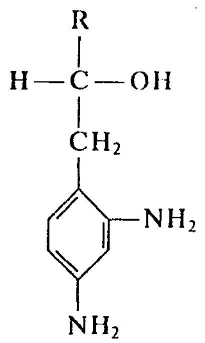

in der R ein Wasserstoffatom, eine $CH_3$- oder $C_2H_5$-Gruppe bedeutet, auch in Form seiner anorganischen oder organischen Salze, enthält.

2. Mittel nach Anspruch 1, dadurch gekennzeichnet, daß es als Kupplersubstanz 1-(β-Hydroxyäthyl)-2,4-diaminobenzol, auch in Form seiner anorganischen oder organischen Salze, enthält.

3. Mittel nach Anspruch 1 und 2, dadurch gekennzeichnet, daß es die erfindungsgemäßen Kupplersubstanzen in einer Konzentration von 0,02 bis 2,0 Gewichtsprozent enthält.

4. Mittel nach Anspruch 1 bis 3, dadurch gekennzeichnet, daß es mindestens eine der Entwicklersubstanzen, 1,4-Diaminobenzol, 2,5-Diaminotoluol, 2,5-Diaminobenzylalkohol, p-Aminophenol und 3-Methyl-4-aminophenol enthält.

5. Mittel nach Anspruch 1 bis 4, dadurch gekennzeichnet, daß es zusätzlich mindestens eine der Kupplersubstanzen α-Naphthol, 3,4-Diaminobenzoesäure, Resorcin, 4-Chlorresorcin, m-Aminophenol, 4-Oxy-1,2-methylendioxybenzol und 3-Amino-6-methylphenol enthält.

6. Mittel nach Anspruch 1 bis 5, dadurch gekennzeichnet, daß die Gesamtmenge der enthaltenen Entwickler-Kupplersubstanz-Kombination 0,5 bis 3,0 Gewichtsprozent beträgt.

7. Mittel nach Anspruch 1 bis 6, dadurch gekennzeichnet, daß es zusätzlich als Farbkomponente 6-Amino-3-methylphenol enthält.

8. Mittel nach Anspruch 1 bis 7, dadurch gekennzeichnet, daß es zusätzlich mindestens einen der direktziehenden Farbstoffe Diamond Fuchsin (C.I. 42 510), Leather Ruby HF (C.I. 42 520), 2-Nitro-1,4-diaminobenzol, 2-Amino-4-nitrophenol, Acid Brown 4 (C.I. 14 805), Acid Blue 135 (C.I. 13 385), Disperse Red 15 (C.I. 60 710), Disperse Violet 1 (C.I. 61 100), 1,4,5,8-Tetraamino-anthrachinon und 1,4-Diaminoanthrachinon enthält.

9. Mittel nach Anspruch 1 bis 8, dadurch gekennzeichnet, daß es zusätzlich Antioxidantien, vorzugsweise Ascorbinsäure oder Natriumsulfit, enthält.

10. Mittel nach Anspruch 1 bis 9, dadurch gekennzeichnet, daß es zusätzlich Netzmittel, Emulgatoren und/oder Verdicker enthält.

11. Verfahren zur oxidativen Färbung von Haaren, dadurch gekennzeichnet, daß man ein, eine Kombination von in der Haarfärbung bekannten Entwicklersubstanzen mit einem 1-(β-Hydroxyalkyl)-2,4-diaminobenzol der allgemeinen Formel

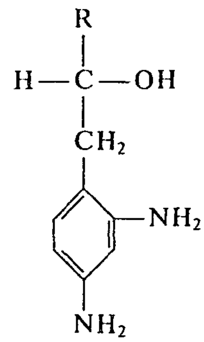

in der R ein Wasserstoffatom, eine $CH_3$- oder $C_2H_5$-Gruppe bedeutet, auch in Form seiner Salze mit anorganischen oder organischen Säuren, als Kupplersubstanz sowie gegebenenfalls zusätzlich bekannte Kupplersubstanzen enthaltendes, Haarfärbemittel gemäß den Ansprüchen 1 bis 10 nach Zusatz eines Oxidationsmittels, insbesondere Wasserstoffperoxid, auf die Haare aufbringt, 10 bis 45 Minuten lang bei einer Temperatur von 15 bis 50° C einwirken läßt, anschließend die Haare spült, gegebenenfalls wäscht und nachspült, und sodann trocknet.

**Claims**

1. Product for the oxidative dyeing of hair with a content of from 0.1 to 5.0 weight percent of a developer substance-coupler substance combination, characterised in that it contains as coupler substance 0.01 to 4.0 weight percent of a 1-(β-hydroxyalkyl)-2,4-diaminobenzene having the general formula

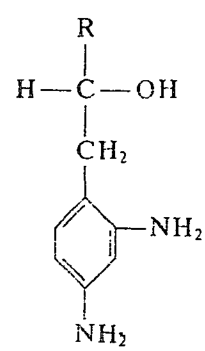

in which R is a hydrogen atom, a $CH_3$ or $C_2H_5$ group, and which may also be in the form of inorganic or organic salts.

2. Product according to Claim 1, characterised in that it contains as coupler substance 1-(β-hydroxyethyl)-2,4-diaminobenzene, which may also be in the form of inorganic or organic salts.

3. Product according to Claim 1 and Claim 2, characterised in that it contains the coupler substances according to the invention in a concentration from 0.02 to 2.0 weight percent.

4. Product according to any of claims 1 to 3,

characterised in that it contains at least one of the developer substances 1,4-diaminobenzene, 2,5-diaminotoluene, 2,5-diaminobenzyl alcohol, p-aminophenol and 3-methyl-4-aminophenol.

5. Product according to any of claims 1 to 4, characterised in that it additionally contains at least one of the coupler substances α-naphthol, 3,4-diaminobenzoic acid, resorcinol, 4-chlororesorcinol, m-aminophenol, 4-oxy-1,2-methylenedioxybenzene and 3-amino-6-methylphenol.

6. Product according to any of claims 1 to 5, characterised in that the total quantity of the developer-coupler substance combination amounts to 0.5 to 3.0 weight percent.

7. Product according to any of claims 1 to 6, characterised in that it additionally contains as colouring component 6-amino-3-methylphenol.

8. Product according to any of claims 1 to 7, characterised in that it additionally contains at least one of the direct dyes Diamond Fuchsin (C.I. 42 510), Leather Ruby HF (C.I. 42 520), 2-nitro-1,4-diaminobenzene, 2-amino-4-nitrophenol Acid Brown 4 (C.I. 14 805), Acid Blue 135 (C.I. 13 385), Disperse Red 15 (C.I. 60 710), Disperse Violet 1 (C.I. 61 100), 1,4,5,8-tetraaminoanthraquinone and 1,4-diamino-anthraquinone.

9. Product according to any of claims 1 to 8, characterised in that it additionally contains antioxidants, preferably ascorbic acid or sodium sulphite.

10. Product according to any of claims 1 to 9, characterised in that it additionally contains wetting agents, emulsifiers and/or thickeners.

11. Process for the oxidative dyeing of hair, characterised in that there is applied to the hair a hair dyeing product according to any of claims 1 to 10 containing a combination of developer substances previously known in the hair colouring art with, as coupler substances, 1 1-(β-hydroxyalkyl)-2,4-diaminobenzene of the general formula:

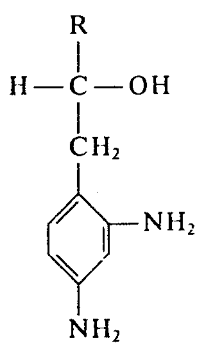

in which R is a hydrogen atom, a $CH_3$ or $C_2H_5$ group, and which may also be in the form of salts with inorganic or organic acids, and, optionally, previously known coupler substances, the hair dyeing product being applied after addition of an oxidative medium, especially hydrogen peroxide, it is allowed to act for 10 to 45 minutes at a temperature of 15 to 50° C, the hair is then rinsed, optionally washed and rinsed again, and then dried.

**Revendications**

1. Produit pour la teinture par oxydation des cheveux ayant une teneur de 0,1 à 5,0 en poids d'une combination substance de développement-substance de copulation ou condensation, caractérisée en ce qu'elle renferme de 0,01 à 4,0% en poids d'un 1-(β-hydroxyalcoyl)-2,4-diaminobenzène de formule générale:

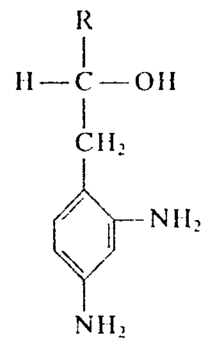

dans laquelle R est un atome d'hydrogène ou un groupe $CH_3$ ou $C_2H_5$, également sous forme de ses sels inorganiques ou organiques, à titre de substance de copulation ou condensation.

2. Produit suivant la revendication 1, caractérisée en ce qu'elle renferme, comme substance de condensation, du 1-(β-hydroxyéthyl)-2,4-diaminobenzène, également sous forme de ses sels inorganiques ou organiques.

3. Produit suivant la revendication 1 ou 2, caractérisée en ce qu'elle renferme les substances de condensation suivant l'invention selon une concentration de 0,02 à 2,0% en poids.

4. Produit suivant l'une quelconque des revendications 1 à 3, caractérisée en ce qu'elle renferme l'une au moins des substances de développement suivantes: 1,4-diaminobenzène, 2,5-diaminotoluène, alcool 2,5-diaminobenzylique, p-aminophénol et 3-méthyl-4-aminophénol.

5. Produit suivant l'une quelconque des revendications 1 à 4, caractérisée en ce qu'elle renferme en outre l'une au moins des substances de condensation suivantes: α-naphtène, acide 3,4-diaminobenzoique, résorcine, 4-chlororésorcine, m-aminophénol, 4-oxy-1,2-méthylènedioxybenzène et 3-amino-6-méthylphénol.

6. Produit suivant l'une quelconque des revendications 1 à 5, caractérisée en ce que la quantité totale de combination substance de développement-substance de condensation qu'elle renferme représente de 0,5 à 3,0% en poids.

7. Produit suivant l'une quelconque des revendications 1 à 6, caractérisée en ce qu'elle renferme en outre, comme constituant formant colorant, du 6-amino-3-méthylphénol.

8. Produit suivant l'une quelconque des revendications 1 à 7, caractérisée en ce qu'elle renferme en outre l'un au moins des colorants à effet direct suivants: Diamond Fuchsine (C.I. 42 510), Leather Ruby HF (C.I. 42 520), 2-nitro-1,4-diaminobenzène, 2-amino-4-nitrophénol, Acide Brown 4 (C.I. 14 805), Acide Blue 135 (C.I. 13 385),

Disperse Red 15 (C.I. 60 710), Disperse Violet 1 (C.I. 61 100), 1,4,5,8-tétraamino-anthraquinone et 1,4-diamino-anthraquinone.

9. Produit suivant l'une quelconque des revendications 1 à 8, caractérisée en ce qu'elle renferme en outre des antioxydants, de préférence de l'acide ascorbique ou du sulfite de sodium.

10. Produit suivant l'une quelconque des revendications 1 à 9, caractérisée en ce qu'elle renferme en outre des agents mouillants, des émulsionnants et (ou) des épaississants.

11. Procédé pour la teinture par oxydation des cheveux, caractérisé en ce qu'on applique sur les cheveux un produit pour la teinture des cheveux renfermant une combination de substances de développement connues pour la teinture des cheveux avec un 1-(β-hydroxyalcoyl)-2,4-diaminobenzène de formule générale:

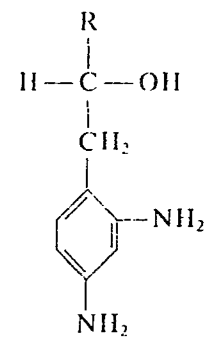

dans laquelle R est un atome d'hydrogène ou un groupe $CH_3$ ou $C_2H_5$, également sous forme de ses sels avec des acides inorganiques ou organiques, à titre de substance de condensation, ainsi que le cas échéant, de façon additionnelle, des substances de condensation connues, suivant l'une quelconque des revendications 1 à 10, après addition d'un agent oxydant, en particulier de peroxyde d'hydrogène, on laisse agir 10 à 45 minutes à une température de 15 à 50°C, puis on rince les cheveux, éventuellement on les lave et on les rince à nouveau, et ensuite on les sèche.